# EUROPEAN PATENT SPECIFICATION

(11) **EP 4 469 792 B1**
(45) Date of publication and mention of the grant of the patent: **04.02.2026**
(21) Application number: 23702310.6
(22) Date of filing: 26.01.2023
(51) Int. Cl.: G01N 22/04, G01N 33/24

(54) **MEASUREMENT APPARATUS FOR AND MEASUREMENT METHOD OF MEASURING MOISTURE OF MINERAL MATERIAL**
MESSVORRICHTUNG UND MESSVERFAHREN ZUR MESSUNG DER FEUCHTIGKEIT VON MINERALISCHEM MATERIAL
APPAREIL ET PROCÉDÉ DE MESURE D'HUMIDITÉ DE MATIÈRE MINÉRALE

(30) Priority: 28.01.2022 FI 20225076
(43) Date of publication of application: 04.12.2024
(73) Proprietor: Senfit Oy, 90590 Oulu (FI)
(72) Inventor: JAKKULA, Pekka, 90590 Oulu (FI); VUOLTEENAHO, Mikko, 90590 Oulu (FI); FISK, Vesa, 90590 Oulu (FI)
(74) Representative: Kolster Oy Ab
(86) International application number: PCT/EP2023/051893
(87) International publication number: WO 2023/144250

(56) References cited:
- WO-A1-2014/076506
- CN-U- 203 490 183
- US-A1- 2012 056 627
- HUANG ET AL: "Microwave cavity perturbation technique for measuring the moisture content of sulphide minerals concentrates", MINERALS ENGINEERING, ELSEVIER, AMSTERDAM, NL, vol. 20, no. 1, 29 November 2006 (2006-11-29), pages 92 - 94, XP005783396, ISSN: 0892-6875, DOI: 10.1016/J.MINENG.2006.04.014
- MILJAK D. G. ET AL: "On-Line Microwave Moisture Measurement of Iron Ore and Mineral Concentrates in Conveyor Applications", 2013 IEEE INTERNATIONAL INSTRUMENTATION AND MEASUREMENT TECHNOLOGY CONFERENCE (I2MTC), 12 March 2007 (2007-03-12), pages 183 - 186, XP093033152, ISSN: 1091-5281, ISBN: 978-1-4673-4621-4, DOI: 10.1109/IMTC.2006.328366
- GAO FENG: "On-Line Measurement of Moisture Content of Iron Ore Slurries", DULUTH JOURNAL OF UNDERGRADUATE RESEARCH, 28 July 2014 (2014-07-28), XP093033155, Retrieved from the Internet <URL:https://conservancy.umn.edu/handle/11299/187021> [retrieved on 20230320]

## Description

### Field

The invention relates to a measurement apparatus for and a measurement method of measuring moisture of mineral material.

### Background

Moisture measurement of non-magnetic mineral material is commonly measured using radio frequency electromagnetic radiation. A drawback of this method is that the measurement of moisture percentage of the material depends on the magnetic properties of the material. However, relative magnetic permeability and magnetic losses of, for example, Magnetite mineral are high and varying. Hence, when electromagnetic radiation of the radio frequency is transmitted through such a mineral sample, the moisture measurement is not reliable because of the random variation of permeability and magnetic losses. Additionally, the moisture measurement is sensitive to the material density, which may also vary randomly. Document Study of the Magnetite to maghemite transition using microwave permittivity and permeability, J. A. Cuenca et aI., 16.2.2016 presents a microwave cavity perturbation measurement.

US 2012/056627 Al discloses moisture measurements of soil using a cavity filled with a high-permittivity material.

These problems have been identified but no useful solution for a proper moisture measurement exists. There have been attempts to overcome the problem using a reflection measurement instead. The reflection measurement is not accurate because it is sensitive to the surface roughness, slope, reflections from environment and the material density, it is affected by material permeability, and it is not representative (only surface moisture is measured). Hence, an improvement would be welcome.

### Brief description

The present invention seeks to provide an improvement in the measurements.

The invention is defined by the independent claims. Embodiments are defined in the dependent claims.

If one or more of the embodiments is considered not to fall under the scope of the independent claims, such an embodiment is or such embodiments are still useful for understanding features of the invention.

### List of drawings

Example embodiments of the present invention are described below, by way of example only, with reference to the accompanying drawings, in which
Figure 1 illustrates an example of a measurement apparatus;
Figure 2 illustrates an example of the solid material structure with a probe arrangement;
Figure 3 illustrates an example of feeding systems;
Figure 4 illustrates an example of locations of probes;
Figure 5 illustrates an example of feeding probes;
Figure 6 illustrates an example of another kind of resonator;
Figure 7 illustrates an example of a frequency response of a resonator;
Figure 8 illustrates an example of the data processing unit;
Figure 9 illustrates an example of tilted disturbing resonance; and
Figure 10 illustrates of an example of a flow chart of a measuring method.

### Description of embodiments

The following embodiments are only examples. Although the specification may refer to "an" embodiment in several locations, this does not necessarily mean that each such reference is to the same embodiment(s), or that the feature only applies to a single embodiment.

The articles "a" and "an" give a general sense of entities, structures, components, compositions, operations, functions, connections or the like in this document. Note also that singular terms may include pluralities.

Single features of different embodiments may also be combined to provide other embodiments. Furthermore, words "comprising" and "including" should be understood as not limiting the described embodiments to consist of only those features that have been mentioned and such embodiments may also contain features/structures that have not been specifically mentioned. All combinations of the embodiments are considered possible if their combination does not lead to structural or logical contradiction.

The term "about" means that quantities or any numeric values are not exact and typically need not be exact. The reason may be tolerance, resolution, measurement error, rounding off or the like, or a fact that the feature of the solution in this document only requires that the quantity or numeric value is approximately that large. A certain tolerance is always included in real life quantities and numeric values.

It should be noted that while Figures illustrate various embodiments, they are simplified diagrams that only show some structures and/or functional entities. The connections shown in the Figures may refer to one or more similar or different connections. It is apparent to a person skilled in the art that the described apparatus may also comprise other functions and structures than those described in Figures and text. It should be appreciated that details of some functions, structures, and the signalling used for measurement and/or controlling are irrelevant to the actual invention. Therefore, they need not be discussed in more detail here.

Magnetite material such as Magnetite concentrate and Magnetite ore have proven difficult for the prior art measurements based on the standard microwave transmission technology. The reason for the difficulty is believed to be in the magnetic nature or varying magnetic permeability of Magnetite material. The magnetic permeability is the degree of magnetization of a material to a magnetic field.

It has also been considered that the microwave or radiofrequency transmission signal is largely affected by the water within ore or Magnetite material as well as by the magnetic permeability of ore or Magnetite material. This is a problem because the moisture changes cannot then be distinguished from the magnetic permeability changes.

It is known that in microwave transmission the electric and magnetic fields are in the same phase. This means that electric and magnetic properties of materials have the same effect on the travelling wave. In resonators the situation is different. In radio frequency and microwave cavity resonators the phase difference between electric and magnetic fields is π/2. This means that in places where the electric field has a maximum the magnetic field has a minimum. From that it can be concluded that if a measured sample of material is located inside a resonator in a place where the electric field is strong and the magnetic field is weak, the moisture of the sample can be measured with the electric field such that moisture result does not depend on the magnetic properties of the material of the sample at all or the dependence is limited and acceptable, which enables a reliable moisture measurement.

The relative density of Magnetite is about 5 which means that a volume of Magnetite weights about 5 times more than the same volume of water (relative density of water is 1). The moisture content of Magnetite material, which is an example of mineral material, is typically about 0% to about 10% in weight in many processes which means about 0% to about 35% in volume. The term mineral material refers to mineral concentrate, which is the valuable portion of the mineral material, or gangue mineral, which is the invaluable portion of the mineral material. The gangue mineral may also be called tailings or waste stream. Any magnetic material can be measured because such material is based on mineral material.

Normally microwave resonator measurements are used at water contents below 30% in volume in the prior art. This means that a special resonator arrangement is needed for measuring mineral material. In order to perform the measurement magnetic field should be cancelled or brought to a minimum in the measurement volume. Simultaneously, it is a benefit if the electric field strength is reduced in the measurement volume and the measured volume inside the resonator is relatively small compared to the total volume of resonator structure. By that way the measurement is adequate for high water contents and for other mineral materials even denser than Magnetite, as well.

It is known that the electric properties of magnetic materials can be measured by using circular waveguide resonator with TM₀₁₀ mode where the magnetic field is zero in the middle of the resonator. The drawback of this method is that the volume where the magnetic field is zero is very small and cannot be used to measure large material volumes as it is required in industrial measurements. This kind of measurement is presented in Study of the Magnetite to maghemite transition using microwave permittivity and permeability measurements, Jerome Alexander Cuenca, Keith Bugler, Stuart Taylor, David Morgan, Paul Williams, Johann Bauer and Adrian Porch, Journal of Physics: Condensed Matter, Volume 28, Number 10, 16 February 2016.

The solution to this problem is to enlarge the volume of magnetic field's zero by filling the resonator partly by a high permittivity material. This method has two effects: First the electric field strength will be higher inside the high permittivity part and lower in the center of the resonator where the material under measurement is located. Secondly the magnetic field's zero volume is increased. Here, the value zero means that the value is zero or at least approximately zero. That a value is at least approximately zero refers to a value that corresponds, within tolerance or resolution of the measurement, to zero, i.e. it does not disturb the measurement significantly but instead a reliable result can be achieved from the measurement.

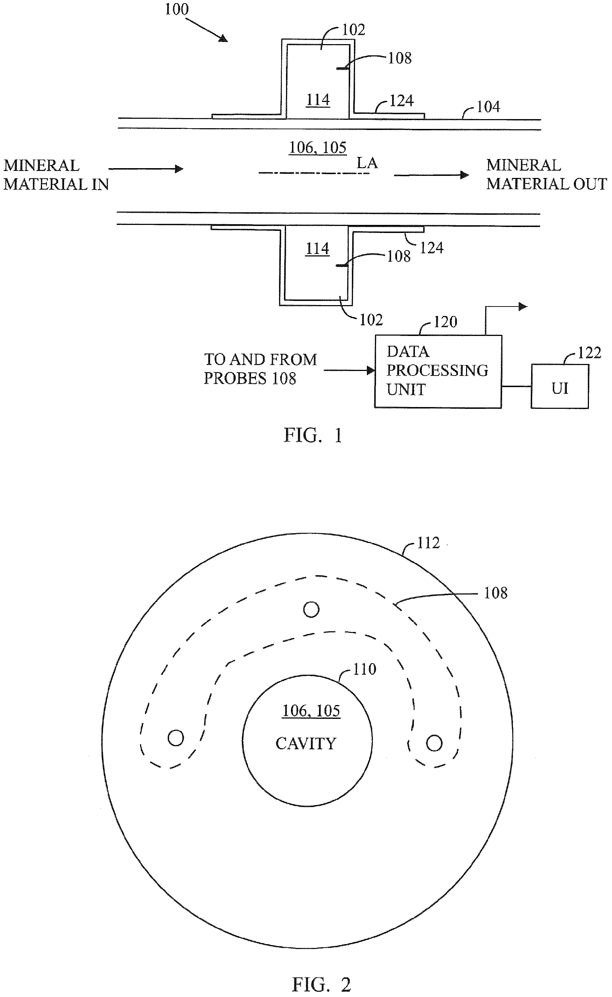

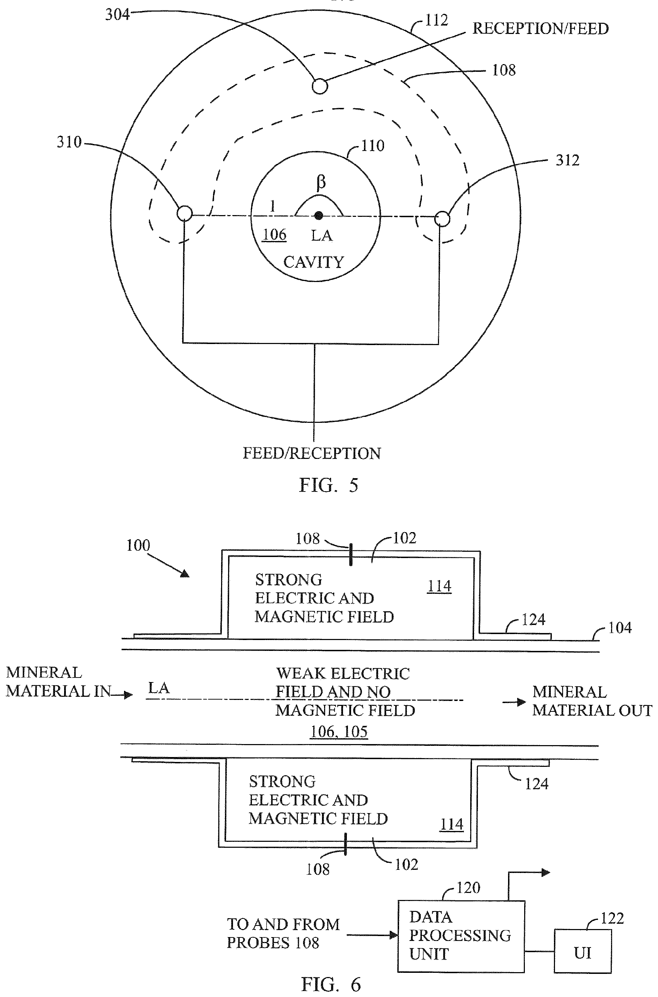

Fig. 1 illustrates an example of a measurement apparatus for measuring moisture of mineral material seen from side such that a longitudinal axis LA of a duct 104 extend from left to right. Fig. 2 illustrates an example of a measurement apparatus seen in the direction of the longitudinal axis LA of the duct 104. The measurement apparatus comprises a resonator 100. The resonator 100 is located round the duct 104. A cross section of the resonator 100 is bounded by and containing an area of a solid material structure 114 between an inner circumference 110 and an outer circumference 112. Alternatively, the resonator 100 may have two parts facing each other at opposite sides of the duct 104 (like they seemingly are in Fig. 1; see also Fig. 6).

The inner and outer circumferences 110, 112 have a distance therebetween which is filled with a solid material. A cavity 106 of the measurement apparatus, which includes a volume where interaction between the electromagnetic radiation and a sample of mineral material takes place, is within the inner circumference 110.

Relative permittivity of the solid material structure 114 of the resonator 100 between the inner circumference 110 and the outer circumference 112 is higher than that of a relative permittivity of the cavity 106 with mineral material. Usually, a range of relative permittivity of the mineral material is known beforehand based on various chemical analyses of the minerals. Hence, it is easy to select the solid material for the resonator 100. On the other hand, if an estimate of the relative permittivity of the mineral material is not known, the relative permittivity of the solid material structure 114 may be selected as high as possible. Then test runs may be performed. In an embodiment, solid material of the solid material structure 114 may comprise Zirconia ZrO₂. The relative permittivity of Zirconia is about 30 whereas the relative permittivity of Magnetite is about 5.2, when measured using frequency about 2.5 GHz.

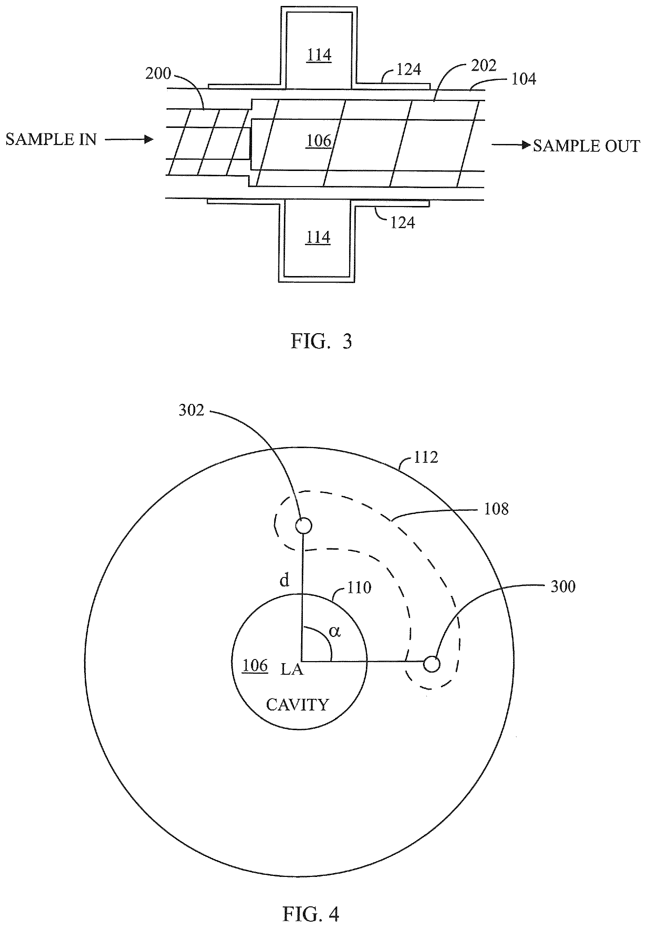

The resonator 100 comprises, within the solid material structure 114, a probe arrangement 108 which is configured to cause an electromagnetic resonance of the resonator 100 within the cavity 106 in response to electric energy fed through the probe arrangement 108 to the resonator 100. In Fig. 2, the probes may be coaxial probes, for example. Each of the probes 300, 302, 304, 310, 312 (see Figs 4, 5) may be considered to comprise an electric conductor, which is inserted into or onto the solid material structure 114. The resonator 100 can, in general, be excited by several methods for example by a loop or a hole, which, *per se,* are known by a person skilled in the art. Because the solid material fills a waveguide or a resonance cavity of the resonator 100 partly, the probes and an external electric circuit, such as a data processing unit 120, are coupled together such that electric energy can be fed to the resonator 100 and a frequency response of the resonator 100 can be measured.

The solid material structure 114 of the resonator 100 and the cavity 106 are configured to cause an uneven distribution of a resonating magnetic field of an electromagnetic field between the cavity 106 and the solid material structure 114. As a result, the cavity 106 has a lower permittivity than the solid material structure 114 of the resonator 100. The solid material structure 114 has higher permittivity than the cavity 106. The magnetic field is cancelled in the cavity 106. The magnetic field has zero field in the middle of the cavity 106 and the high permittivity of material 114 has increased the effective size of the resonator and made magnetic zero volume larger. Hence, this difference in these relative permittivities causes the magnetic field to be mainly located within the solid material structure 114 of the resonator 100 and relatively more reluctant to reside within the cavity 106. A large portion of the cavity 106 is typically air and the solid material structure 114 has mainly material of high relative permittivity. The walls of the cavity 106 are made of solid material of low relative permittivity. A range of the relative permittivity of the walls of the cavity 106 may be 2 to 10, for example. Permittivity is ability of a material to allow the magnetic field to pass through it.

Electric field has normally its maximum in the center of the resonator. However, by placing material of higher permittivity suitably outside the center of the resonator, the electric field will move partly in the direction of higher permittivity and for that reason the electric field is weakened in the volume at the center of the resonator and its adjacency. Based on that, moisture percentage of a variety of materials including those, which are normally challenging to measure, can be measured by placing them in the low electric field at the center of the resonator 100.

Hence, the measurement of moisture content of the mineral material within the cavity 114 is mainly based on the electric field in order to avoid problems related to the magnetic properties of the mineral material.

During a moisture measurement, the cavity 106 contains the mineral material. The measurement of mineral material may be a batch process or a continuous flow process. In the batch process, the cavity 106 may act as a measurement chamber and the steps of the measurement may include filling the measurement chamber with the mineral material fully or partially, performing the measurement, and emptying the measurement chamber. Then a new measurement can be performed starting from the filling of the measurement chamber.

In a continuous flow measurement, the mineral material flows through the cavity 106 continuously and the measurements are performed continuously or repeatedly. The repeated measurements may performed regularly or irregularly.

Fig. 3 illustrates an example of an embodiment, where the measurement apparatus comprises two sample feeding systems 200, 202. A first sample feeding system 200 feeds the mineral material toward a second sample feeding system 202 at a first volumetric flow rate, and the second sample feeding system 202 feeds the mineral material to and through the cavity 106 of the resonator 100 at a second volumetric flow rate. The volumetric flow rate defines the volume of mineral material that passes the cavity 106 in a unit of time. The second volumetric rate may be greater than the first volumetric flow rate. The difference in the volumetric rates i.e. speeds of flow causes the amount of mineral material per time unit to increase with respect to that of the first feeding system 200.

The volumetric flow rate may also be kept constant if the volume of the feeding system 202 is larger than that of the feeding system 200. In that manner, moving part of the feeding systems 200, 202 may be the same but an inner volume of immobile parts i.e. a diameter or a square root of the cross sectional area of the duct 104 may differ such that the diameter or the square root of the cross sectional area is larger within the resonator 100 than outside the resonator 100. However, both the volumetric flow rate and the diameter or the square root of the cross sectional area may be larger within the resonator 100 than outside the resonator 100. Fig. 3 illustrates both the larger cross sectional area of the duct 104 and the volumetric rate within the resonator 100 than outside the resonator 100.

When the cavity 106 is not full of the mineral material, an average relative permittivity of the cavity 106 remains lower than that of the solid material structure 114. That feature enables a measurement of materials of high relative permittivity. That is, permittivity of the mineral material alone may even be higher than that of the solid material structure 114 but an average of the cavity 106 still remains lower. That causes the magnetic field to reside mainly within the solid material structure 114 and enables a proper measurement of moisture percentage of the mineral material. That also causes the electric field to reside mainly within the cavity 106 for enabling a proper measurement of moisture percentage of the mineral material based on electric field.

The first feeding system 200 may be a screw feeder, a screw auger feeder, a conveyer belt, any combination of these or the like. The second feeding system 202 may be a screw feeder, a screw auger feeder, a conveyer belt, any combination of these or the like built from electrically non-conductive material. In the case of screw feeders, the first and the second feeding systems 200, 202 may have a common shaft such that both rotates with the same rotational speed. However, because of a larger diameter of the second feeding system 202 than that of the first feeding system 200, the second feeding system 202 has a larger volumetric flow rate that that of the first feeding system 200. Another solution is that the first and second feeding systems 200, 202 have different rotation speeds.

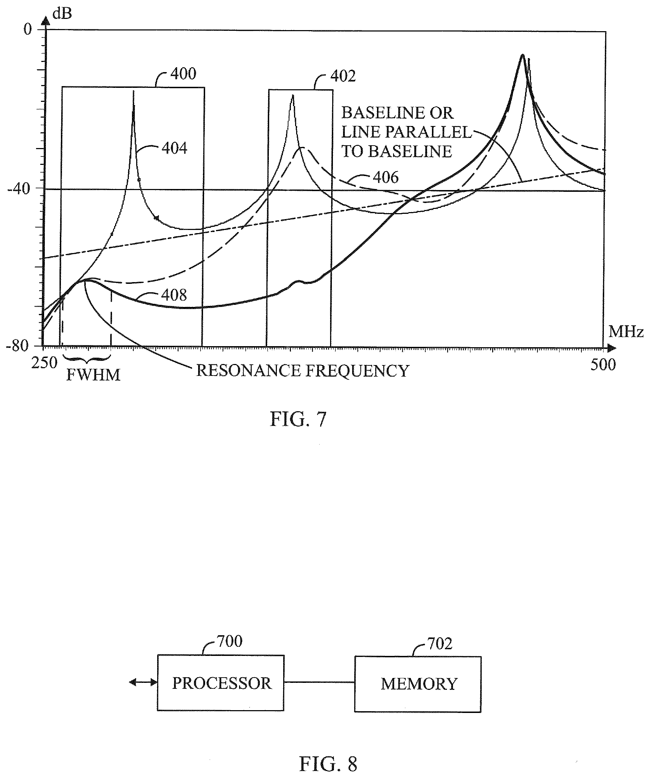

The probe arrangement 108 delivers or outputs information on a frequency response of the resonator 100. The frequency response may include a resonance frequency or a resonance frequency range. The resonance frequency range may include a full width at half maximum (FWHM) or a corresponding width related to the resonance frequency (see example of FWHM in Fig. 7).

The frequency response may include a level of the signal at a resonance frequency or a frequency range (see Fig. 7). The level may refer to amplitude or power of the signal. The level of the resonance caused by the mineral material may be measured with respect to a result measured without the mineral material i.e. without any sample. Alternatively or additionally, the reference may be a measurement with a known and/or predefined sample.

The frequency response may include Q-value of the resonance. The Q-value refers to a quality factor that describes how underdamped or damped oscillation in the resonator is. A person skilled in the art is familiar with the Q-value, *per se.*

These parameters (resonance frequency, level, Q-value) are affected by moisture of the mineral material that is within the cavity 106 for determination of a moisture content of the mineral material. The baseline may also be taken into account (see example of baseline in Fig. 7). In an embodiment, by measuring two resonator parameters the density compensation of the mineral material can be determined. Namely, if the density of the mineral material varies, it also affects the measurement but the effect caused by the density variation can be cancelled with the measurement of two parameters. Such measurement results can be presented with a pair equations which have two variables: density and moisture. Because of the pair of equations, both or either of the variables can be solved. If the density of the measured material is at least approximately constant also one parameter is enough and suitable for measuring moisture of the mineral material.

The density may also be compensated by measuring weight of the mineral material that is measured. The measurement of weight may be continuous or sample by sample.

In an embodiment, an optimal operating frequency of the measurement apparatus may be in the range from about 100 MHz to 1 GHz. At lower frequencies the disturbing electric conductivity effects will rise and at higher frequencies the resonator size will be unpractically small.

In an embodiment, relative permittivity of the solid material structure 114 may be in a range 20 to 100. In this embodiment, the permittivity of the material under measurement is lower than that of the solid material structure 114.

The measurement apparatus may also comprise a data processing unit 120, which determines the moisture content of the mineral material based on the frequency response which may also include the level of the signal of the resonator 100 delivered by the probe arrangement 108. The data processing unit 120 may also feed the electric energy to the resonator 100 through the probe arrangement 108. During a measurement of the mineral material the cavity 106 includes the mineral material, which affects the frequency response of the resonator 100.

In an embodiment an example of which is illustrated in Fig. 8, the data processing unit 120 may comprise one or more processors 700 and one or more memories 702 including computer program code. The one or more memories 702 and the computer program code are configured to, with the one or more processors 700, cause data processing unit at least to determine the moisture content of the mineral material based on the frequency response of the resonator 100 affected by the mineral material.

In an embodiment, the measurement apparatus is configured to measure mineral material, which comprises ferromagnetic and/or ferrimagnetic materials. Alternatively or additionally, the measurement may also be performed when the material includes materials that are paramagnetic and/or diamagnetic.

In an embodiment, the measurement apparatus may measure mineral material, which comprises Magnetite and/or Hematite.

In an embodiment an example of which is illustrated in Fig. 4, the probe arrangement 108 may comprise a second coupling probe 302 located at a position d, α in a polar coordinate system, where d is at a radial distance from a longitudinal axis LA of the resonator 100 of a first feeding probe 300, and the angle α is π/2 between the first probe 300 and the second probe 302. The distance d of probe 302 can be different from probes 300 distance.

In an embodiment an example of which is illustrated in Fig. 5, the second feeding probe 310 may be fed in phase to the first probe 312. In this manner the disturbing resonance 402 can be cancelled because its electric field has an opposite electric field in positions of the feeding probes 310 and 312. Probe 304 is a receiving probe. Resonance 402 is at a frequency different from the measured resonance frequency 400 (see Fig. 7).

Fig. 6 illustrates an example of a resonator which has a cylinder of the solid material structure 114 which has a high relative permittivity material on a duct 104 through which mineral material flows. In this example, the duct 104 is not in the middle a hole of the cylinder of the solid material 114 but instead the cylinders of the solid material 114 are on opposite sides of the duct 104. In this embodiment, like also in the embodiment of illustrated in Fig. 1, the electric field and the magnetic field are strong within the solid material 114. The magnetic field is zero from practical point of view within the duct 104 which enables the measurements of also magnetic materials. The electric field is weaker within the duct 104 that it is within the solid material 114.

Fig. 7 illustrates examples of frequency responses of the resonator 100. The curve 404 illustrates an example of an empty resonator 100 i.e. a measurement without the mineral material. The probe arrangement 108 includes probes at opposite sides of the longitudinal axis LA of the resonator 100. The probes of the probe arrangement 108 may be, for example, monopoles, loops or apertures without limiting to these. In addition to the measurement resonance 404 of the resonator 100, disturbing resonance 402 can be seen clearly. The measurement resonance according to the invention is TM₀₁₀. The disturbing resonance in this example is TM₂₁₀. The third resonance in this example at the right side of Fig. 7 is TM₄₁₀. The curve 406 represents a measurement of mineral material. Also here the measurement resonance 400 can be seen but also the disturbing resonance 402 is present.

In an embodiment an example of which is illustrated in Fig. 5, the probe arrangement 108 may comprise a pair of feeding probes 310, 312 located at a position l, β in a polar coordinate system, where l is at a radial distance from a longitudinal axis LA of the resonator 100 and the angle β is π between the pair of the coupling probes 310, 312. The measurement apparatus is configured to feed the pair of coupling probes 310, 312 a signal that has the same phase for both of them in order to cancel a disturbing resonance 402 at a frequency different from the measured resonance frequency 400. The probe 304 is a receiving probe. The curve 408 in Fig. 7 illustrates an example of the frequency response of moist material of this embodiment, and the disturbing resonance 402 has almost disappeared and the Q-value of the resonance frequency 400 can be measured.

It should also be noted that the effect of the disturbing resonance 402 can alternatively or additionally be eliminated or reduced by the two sample feeding systems 200, 202. The difference in the volumetric rates i.e. speeds of flow causes the amount of mineral material per time unit in the cavity 106 to decrease with respect to a corresponding volume fed by the first feeding system 200. When the cavity 106 is not full of the mineral material, an average relative permittivity of the cavity 106 remains lower than that of the solid material structure 114, even when relative permittivity of mineral material alone is higher than that of the solid material structure 114. That feature enables a measurement of materials of high relative permittivity and reduces the effect of disturbing resonance 402. Alternatively, the probe 304 may be a feeding probe and the reception is performed by the probes 310 and 312.

According to the invention, the solid material structure 114 may be a plate, which has a hole 105 through the plate. The hole 105 is located inside of the outer circumference 112 and the hole 105 comprises the cavity 106. The measurement apparatus may then comprise a duct 104 attached within the hole 105. The duct 104 may extend through the hole 105. In this embodiment, the surface of the cavity 106 is the surface of the duct 104. Alternatively, the duct 104 may comprise two parts. One end of one part of the duct 104 may be fixed concentrically and potentially coaxially to one side of the hole 105 and one end of another part of the duct 104 may be fixed concentrically and potentially coaxially to another side of the hole 105. In this embodiment, the surface of the cavity 106 is the surface of the solid material structure 114.

The duct 104 may allow flow of the mineral material through the measurement apparatus and through the cavity 106.

In an embodiment an example of which is illustrated in Fig. 3, the measurement apparatus may comprise a layer 124 of an electrically conductive material on the solid material structure 114 and on the duct 104 for protecting the measurement from disturbing electromagnetic radiation from the environment and/or preventing the radiating leakage out from the resonator 100.

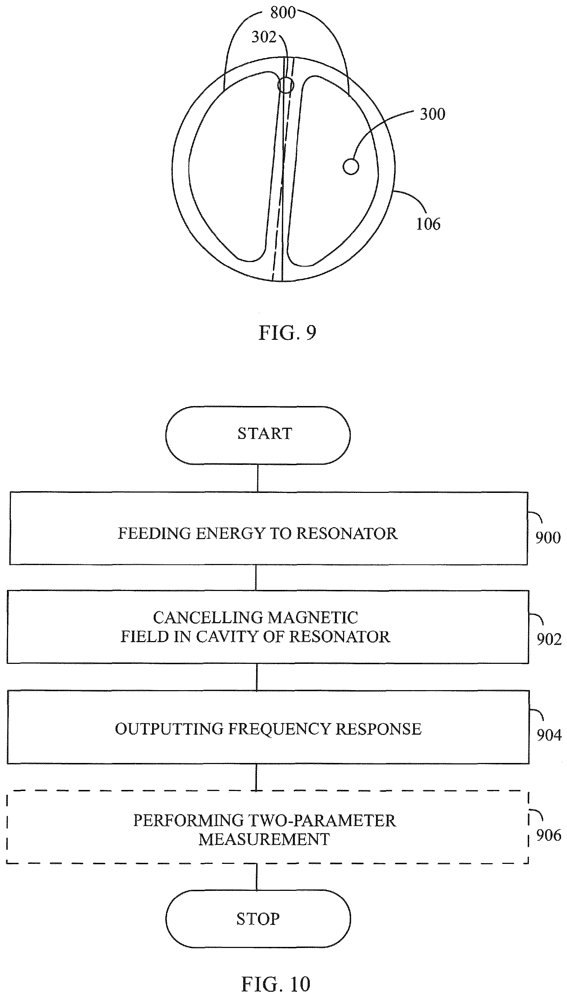

Fig. 9 illustrates an example of rotated electric field 800 in the cavity 106, which may happen. In this case the resonator 100 is fed by probe 312 and received by probe 302 at a location where the minimum electric field of resonance 402 is (a person skilled in the art can easily determine the location of the minimum electric field of the resonance theoretically, based on simulation and/or based on a test). The rotated electric field 800 may cause disturbing resonance shown in Fig. 7. That kind of disturbing resonance 402 may be avoided or reduced by the pair of coupling probes 310, 312 that are fed with a signal of the same phase for both of them.

This kind of structure of the measurement apparatus solves problems related to the measurement of Magnetite and high density nonmagnetic material. Explained in simplified manner, magnetic field is zero or close to it in a wide volume inside the resonator. That is, magnetic field strength in the cavity 106 is low. Electric field strength is zero or about zero on the outer side of the solid material structure 114. Effective measurement volume, i.e. the size of the cavity 106 is small compared to the total effective volume of the whole resonator structure which includes the solid material structure 114 and the cavity 106. A physical diameter of the measurement apparatus may be about 200 mm, for example, and an effective diameter about 1 m, for example. This size allows a representing measurement also in industrial applications.

In an embodiment, at least part of the particles that are measured with the measurement apparatus may be larger than about 0.1 mm and smaller than about 5 mm. In an embodiment, at least part of the particles that are measured with the measurement apparatus may be larger than about 1 mm and smaller than about 5 mm. In an embodiment, at least part of the particles that are measured with the measurement apparatus may be larger than about 2 mm and smaller than about 5 mm. In an embodiment, the flow of the mineral material through the resonator 100 of the measurement apparatus may be about 10 kg to about 50 kg per minute.

Fig. 10 is a flow chart of the measurement method. In step 900, electric energy is fed to a resonator 100 of a measurement apparatus by a probe arrangement 108, which is located within the solid material structure 114 and causes an electromagnetic resonance within a cavity 106 of the resonator 100 in response to the electric energy, the cavity 106 of the measurement apparatus being limited by the solid material structure 114, and a relative permittivity of the solid material structure 114 of the resonator 100 is higher than that of the mineral material measured.

In step 902, an uneven distribution of a magnetic field of the resonance between the cavity 106 and the solid material structure 114 is caused by the solid material structure 114 of the resonator 100 and the cavity 106, and magnetic field in the cavity 106 is cancelled resulting in a distribution where the magnetic field is mainly located within the solid material structure 114 of the resonator 100 and less within the cavity 106.

In step 904 a frequency response of the resonator 100 affected by the mineral material within the cavity 106 is output, by the probe arrangement 108, for determination of a moisture content of the mineral material.

In an optional step 906, a density compensated moisture content of the mineral material is determined based on at least two of the following of the frequency response: a resonance frequency of the resonator 100, a Q-value of the resonance and a level of the signal of the resonance.

The method shown in Fig. 10 may be implemented as a logic circuit solution or computer program. The computer program may be placed on a computer program distribution means for the distribution thereof. The computer program distribution means is readable by a data processing device, and it encodes the computer program commands, carries out the measurements and optionally controls the processes on the basis of the measurements.

The computer program may be distributed using a distribution medium which may be any medium readable by the controller. The medium may be a program storage medium, a memory, a software distribution package, or a compressed software package. In some cases, the distribution may be performed using at least one of the following: a near field communication signal, a short distance signal, and a telecommunications signal.

It will be obvious to a person skilled in the art that, as technology advances, the inventive concept can be implemented in various ways. The invention and its embodiments are not limited to the example embodiments described above but may vary within the scope of the claims.

## Claims

1. A measurement apparatus for measuring moisture of mineral material, and the measurement apparatus comprises a resonator (100), **characterized in that**
the resonator (100), which is a radio and/or microwave cavity resonator, is partly filled with a solid material structure (114);
a relative permittivity of the solid material structure (114) of the resonator (100) is configured to be higher than that of a cavity (106) of the resonator with mineral material;
the resonator (100) comprises, within the solid material structure (114), a probe arrangement (108) which is configured to cause an electromagnetic resonance of the resonator (100) within the cavity (106) in response to electric energy fed through the probe arrangement (108) to the resonator (100) with TM₀₁₀ mode;
the solid material structure (114) of the resonator (100) and the cavity (106) are configured to cause an uneven distribution of a magnetic field of the resonance between the cavity (106) and the solid material structure (114) such that a magnetic field is cancelled in the cavity (106) caused by a higher relative permittivity of the solid material structure (114) than that of the cavity (106);
the solid material structure (114) is a plate, which has a hole (105) through the plate, and the hole (105) is located inside the outer circumference (112) and the hole (105) comprises the cavity (106);
the cavity (106) is configured to contain the mineral material for a moisture measurement; and
the probe arrangement (108) is configured output a frequency response of the resonator (100) affected by the mineral material for determination of moisture content of the mineral material.

2. The measurement apparatus of claim 1, **characterized in that** the solid material structure (114) of the resonator (100) comprises Zirconia ZrO₂.

3. The measurement apparatus of claim 1, **characterized in that** the measurement apparatus comprises a data processing unit (120), which is configured to determine the density compensated moisture content of the mineral material based on the frequency response of the resonator (100) affected by the mineral material.

4. The measurement apparatus of claim 3, **characterized in that** the data processing unit (120) is configured to determine the density compensated moisture content of the mineral material based on at least two of the following of the frequency response: a resonance frequency of the resonator (100), a Q-value of the resonance and a signal level of the resonance.

5. The measurement apparatus of claim 3, **characterized in that**
the data processing unit (120) comprises one or more processors (700); and one or more memories (702) including computer program code;
the one or more memories (702) and the computer program code configured to, with the one or more processors (700), cause data processing unit (120) at least to:
determine the moisture content of the mineral material based on the frequency response of the resonator (100) affected by the mineral material.

6. The measurement apparatus of claim 1, **characterized in that** the measurement apparatus comprises two sample feeding systems (200, 202), a first sample feeding system (200) of which is configured to feed the mineral material toward a second sample feeding system (202) at a first volumetric flow rate, and the second sample feeding system (202) is configured feed the mineral material to and through the cavity (106) of the resonator (100) at a second volumetric flow rate, which is greater than the first volumetric flow rate.

7. The measurement apparatus of claim 1, **characterized in that** the measurement apparatus is configured to measure mineral material, which comprises ferromagnetic and/or ferrimagnetic materials.

8. The measurement apparatus of claim 1 or 7, **characterized in that** the measurement apparatus is configured to measure mineral material, which comprises Magnetite and/or Hematite.

9. The measurement apparatus of claim 1, **characterized in that** the probe arrangement (108) comprises a second coupling probe (302) located at a position (d, α), where d is at a radial distance from a longitudinal axis (LA) of the resonator (100) of a first coupling probe (300), and the angle α is π/2 between the first probe (300) and the second probe (302).

10. The measurement apparatus of claim 9, **characterized in that** the second probe (302) is in the zero of a disturbing resonance (402) in the resonator (100) in order to cancel the disturbing resonance (402) at a frequency different from the measured resonance frequency (400).

11. The measurement apparatus of claim 1, **characterized in that** the probe arrangement (108) comprises a pair of coupling probes (310, 312) located at a position (l, β), where l is at a radial distance from a longitudinal axis of the resonator (100) for the pair of the coupling probes (310, 312) and the angle β is π between the pair of the coupling probes (310, 312), and measurement apparatus is configured to feed the pair of coupling probes (310, 312) with a signal that has the same phase for both of them in order to cancel a disturbing resonance (402) at a frequency different from the measured resonance frequency (400).

12. The measurement apparatus of claim 1, **characterized in that**
the measurement apparatus comprises a duct (104) attached with the hole (105), the duct (104) being configured allow flow of the mineral material through the measurement apparatus;
the measurement apparatus comprises a layer (124) of electrically conductive material on the plate of the solid material structure (114) and the duct (104).

13. A measurement method of measuring moisture of mineral material, the method comprising
feeding (900) electric energy to a resonator (100) of a measurement apparatus by a probe arrangement (108), **characterized in that** the resonator (100), which is a radio and/or microwave cavity resonator, is partly filled with a solid material structure (114), the solid material structure (114) being a plate having a hole (105) through the plate, the hole (105) being located inside the outer circumference (112) and the hole (105) comprising the cavity (106), and the probe arrangement (108) located within the solid material structure (114) causes an electromagnetic resonance within a cavity (106) of the resonator (100) in response to the electric energy fed thereto, the cavity 106 of the measurement apparatus being limited by the solid material structure 114, and a relative permittivity of the solid material structure (114) of the resonator (100) is higher than that of the cavity (106) with mineral material;
causing (902), by the solid material structure (114) of the resonator (100) and the cavity (106), an uneven distribution of a magnetic field of the resonance between the cavity (106) and the solid material structure (114), and cancelling magnetic field in the cavity (106) resulting in a distribution where the magnetic field is mainly located within the solid material structure (114) of the resonator (100) and less within the cavity (106);
outputting (904), by the probe arrangement (108), a frequency response of the resonator (100) affected by the mineral material within the cavity (106) for determination of a moisture content of the mineral material.

14. The measurement method of claim 13, **characterized by** determining (906) a density compensated moisture content of the mineral material based on at least two of the following of the frequency response: a resonance frequency of the resonator (100), a Q-value of the resonance and a level of the signal of the resonance.

## Patentansprüche

1. Messvorrichtung zur Messung der Feuchtigkeit von mineralischem Material, wobei die Messvorrichtung einen Resonator (100) umfasst, **dadurch gekennzeichnet, dass**
der Resonator (100), der ein Radio- und/oder Mikrowellen-Hohlraumresonator ist, teilweise mit einer festen Materialstruktur (114) gefüllt ist;
eine relative Permittivität der festen Materialstruktur (114) des Resonators (100) dazu ausgestaltet ist, höher als diejenige eines Hohlraums (106) des Resonators mit mineralischem Material zu sein;
der Resonator (100) innerhalb der festen Materialstruktur (114) eine Sondenanordnung (108) umfasst, die dazu ausgestaltet ist, als Reaktion auf elektrische Energie, die durch die Sondenanordnung (108) in den Resonator (100) mit TM₀₁₀-Modus eingespeist wird, eine elektromagnetische Resonanz des Resonators (100) innerhalb des Hohlraums (106) zu bewirken;
die feste Materialstruktur (114) des Resonators (100) und der Hohlraum (106) dazu ausgestaltet sind, eine ungleichmäßige Verteilung eines Magnetfeldes der Resonanz zwischen dem Hohlraum (106) und der festen Materialstruktur (114) zu bewirken, derart dass in dem Hohlraum (106) ein Magnetfeld aufgehoben wird, das von einer höheren relativen Permittivität der festen Materialstruktur (114) als diejenige des Hohlraums (106) verursacht wird;
die feste Materialstruktur (114) eine Platte ist, die ein Loch (105) durch die Platte aufweist, und das Loch (105) sich im Inneren des äußeren Umfangs (112) befindet und das Loch (105) den Hohlraum (106) umfasst;
der Hohlraum (106) dazu ausgestaltet ist, das mineralische Material für eine Feuchtigkeitsmessung zu enthalten; und
die Sondenanordnung (108) dazu ausgestaltet ist, einen Frequenzgang des Resonators (100), der von dem mineralischen Material beeinflusst wird, zur Bestimmung eines Feuchtigkeitsgehalts des mineralischen Materials auszugeben.

2. Messvorrichtung nach Anspruch 1, **dadurch gekennzeichnet, dass** die feste Materialstruktur (114) des Resonators (100) Zirconiumdioxid ZrO₂ umfasst.

3. Messvorrichtung nach Anspruch 1, **dadurch gekennzeichnet, dass** die Messvorrichtung eine Datenverarbeitungseinheit (120) umfasst, die dazu ausgestaltet ist, einen dichtekompensierten Feuchtigkeitsgehalt des mineralischen Materials auf der Grundlage des Frequenzgangs des Resonators (100), der von dem mineralischen Material beeinflusst wird, zu bestimmen.

4. Messvorrichtung nach Anspruch 3, **dadurch gekennzeichnet, dass** die Datenverarbeitungseinheit (120) dazu ausgestaltet ist, den dichtekompensierten Feuchtigkeitsgehalt des mineralischen Materials auf der Grundlage von mindestens zwei der folgenden des Frequenzgangs zu bestimmen: einer Resonanzfrequenz des Resonators (100), einem Q-Wert der Resonanz und einem Signalpegel der Resonanz.

5. Messvorrichtung nach Anspruch 3, **dadurch gekennzeichnet, dass**
die Datenverarbeitungseinheit (120) einen oder mehrere Prozessoren (700); und einen oder mehrere Speicher (702) umfasst, die Computerprogrammcode umfassen;
der eine oder die mehreren Speicher (702) und der Computerprogrammcode dazu ausgestaltet sind, mit dem einen oder den mehreren Prozessoren (700) die Datenverarbeitungseinheit (120) zu mindestens Folgendem zu veranlassen:
Bestimmen des Feuchtigkeitsgehalts des mineralischen Materials auf der Grundlage des Frequenzgangs des Resonators (100), der von dem mineralischen Material beeinflusst wird.

6. Messvorrichtung nach Anspruch 1, **dadurch gekennzeichnet, dass** die Messvorrichtung zwei Probenzuführungssysteme (200, 202), von denen ein erstes Probenzuführungssystem (200) dazu ausgestaltet ist, das mineralische Material mit einem ersten Volumendurchsatz hin zu einem zweiten Probenzuführungssystem (202) zuzuführen, und das zweite Probenzuführungssystem (202) dazu ausgestaltet ist, das mineralische Material mit einem zweiten Volumendurchsatz, der größer als der erste Volumendurchsatz ist, zu dem und durch den Hohlraum (106) des Resonators (100) zuzuführen.

7. Messvorrichtung nach Anspruch 1, **dadurch gekennzeichnet, dass** die Messvorrichtung dazu ausgestaltet ist, mineralisches Material zu messen, das ferromagnetische und/oder ferrimagnetische Materialien umfasst.

8. Messvorrichtung nach Anspruch 1 oder 7, **dadurch gekennzeichnet, dass** die Messvorrichtung dazu ausgestaltet ist, mineralisches Material zu messen, das Magnetit und/oder Hämatit umfasst.

9. Messvorrichtung nach Anspruch 1, **dadurch gekennzeichnet, dass** die Sondenanordnung (108) eine zweite Kopplungssonde (302) umfasst, die sich an einer Position (d, α) befindet, wobei d ein radialer Abstand von einer Längsachse (LA) des Resonators (100) einer ersten Kopplungssonde (300) ist und der Winkel α zwischen der ersten Sonde (300) und der zweiten Sonde (302) π/2 beträgt.

10. Messvorrichtung nach Anspruch 9, **dadurch gekennzeichnet, dass** die zweite Sonde (302) sich an der Nullstelle einer störenden Resonanz (402) in dem Resonator (100) befindet, um die störende Resonanz (402) bei einer Frequenz, die sich von der gemessenen Resonanzfrequenz (400) unterscheidet, zu unterdrücken.

11. Messvorrichtung nach Anspruch 1, **dadurch gekennzeichnet, dass** die Sondenanordnung (108) ein Paar Kopplungssonden (310, 312) umfasst, die sich an einer Position (l, β) befinden, wobei 1 ein radialer Abstand von einer Längsachse des Resonators (100) für das Paar Kopplungssonden (310, 312) ist und der Winkel β zwischen dem Paar Kopplungssonden (310, 312) Π beträgt, und die Messvorrichtung dazu ausgestaltet ist, in das Paar Kopplungssonden (310, 312) ein Signal einzuspeisen, das für beide davon die gleiche Phase aufweist, um eine störende Resonanz (402) bei einer Frequenz zu unterdrücken, die sich von der gemessenen Resonanzfrequenz (400) unterscheidet.

12. Messvorrichtung nach Anspruch 1, **dadurch gekennzeichnet, dass**
die Messvorrichtung einen Kanal (104) umfasst, der an dem Loch (105) angebracht ist, wobei der Kanal (104) dazu ausgestaltet ist, Strömung des mineralischen Materials durch die Messvorrichtung zu erlauben;
die Messvorrichtung eine Schicht (124) aus elektrisch leitfähigem Material auf der Platte der festen Materialstruktur (114) und dem Kanal (104) umfasst.

13. Messverfahren zur Messung von Feuchtigkeit von mineralischem Material, wobei das Verfahren umfasst
Einspeisen (900) elektrischer Energie in einen Resonator (100) einer Messvorrichtung durch eine Sondenanordnung (108), **dadurch gekennzeichnet, dass** der Resonator (100), der ein Radio- und/oder Mikrowellen-Hohlraumresonator ist, teilweise mit einer festen Materialstruktur (114) gefüllt ist, wobei die feste Materialstruktur (114) eine Platte ist, die ein Loch (105) durch die Platte aufweist, wobei das Loch (105) sich im Inneren des äußeren Umfangs (112) befindet und das Loch (105) den Hohlraum (106) umfasst, und die Sondenanordnung (108), die sich innerhalb der festen Materialstruktur (114) befindet, als Reaktion auf die elektrische Energie, die dort eingespeist wird, eine elektromagnetische Resonanz innerhalb eines Hohlraums (106) des Resonators (100) bewirkt, wobei der Hohlraum (106) der Messvorrichtung durch die feste Materialstruktur (114) begrenzt wird, und eine relative Permittivität der festen Materialstruktur (114) des Resonators (100) höher als diejenige des Hohlraums (106) mit mineralischem Material ist;
Bewirken (902), durch die feste Materialstruktur (114) des Resonators (100) und den Hohlraum (106), einer ungleichmäßigen Verteilung eines Magnetfelds der Resonanz zwischen dem Hohlraum (106) und der festen Materialstruktur (114) und Unterdrücken des Magnetfelds in dem Hohlraum (106), was eine Verteilung ergibt, in der das Magnetfeld sich hauptsächlich innerhalb der festen Materialstruktur (114) des Resonators (100) und weniger innerhalb des Hohlraums (106) befindet;
Ausgeben (904), durch die Sondenanordnung (108), eines Frequenzgangs des Resonators (100), der von dem mineralischen Material innerhalb des Hohlraums (106) beeinflusst wird, zur Bestimmung eines Feuchtigkeitsgehalts des mineralischen Materials.

14. Messverfahren nach Anspruch 13, **gekennzeichnet durch** das Bestimmen (906) eines dichtekompensierten Feuchtigkeitsgehalts des mineralischen Materials auf der Grundlage von mindestens zwei der folgenden des Frequenzgangs: einer Resonanzfrequenz des Resonators (100), einem Q-Wert der Resonanz und einem Pegel des Signals der Resonanz.

## Revendications

1. Appareil de mesure pour mesurer l'humidité d'un matériau minéral, lequel appareil de mesure comprend un résonateur (100), **caractérisé en ce que**
le résonateur (100), qui est un résonateur à cavité d'ondes radio et/ou de micro-ondes, est partiellement rempli d'une structure en matériau solide (114) ;
la permittivité relative de la structure en matériau solide (114) du résonateur (100) est configurée pour être supérieure à celle d'une cavité (106) du résonateur avec un matériau minéral ;
le résonateur (100) comprend, à l'intérieur de la structure en matériau solide (114), un agencement de sondes (108) qui est configuré pour provoquer une résonance électromagnétique du résonateur (100) à l'intérieur de la cavité (106) en réponse à une énergie électrique délivrée par l'intermédiaire de l'agencement de sondes (108) au résonateur (100) en mode TM₀₁₀ ;
la structure en matériau solide (114) du résonateur (100) et la cavité (106) sont configurées pour provoquer une distribution inégale d'un champ magnétique de la résonance entre la cavité (106) et la structure en matériau solide (114) de façon qu'un champ magnétique soit annulé dans la cavité (106) du fait d'une permittivité relative de la structure en matériau solide (114) supérieure à celle de la cavité (106) ;
la structure en matériau solide (114) est une plaque, qui a un trou (105) à travers la plaque, et le trou (105) est situé à l'intérieur de la circonférence extérieure (112) et le trou (105) comprend la cavité (106) ;
la cavité (106) est configurée pour contenir le matériau minéral pour une mesure de l'humidité ; et
l'agencement de sondes (108) est configuré pour délivrer en sortie une réponse en fréquence du résonateur (100) affectée par le matériau minéral pour une détermination de la teneur en humidité du matériau minéral.

2. Appareil de mesure selon la revendication 1, **caractérisé en ce que** la structure en matériau solide (114) du résonateur (100) comprend de la zircone ZrO₂.

3. Appareil de mesure selon la revendication 1, **caractérisé en ce que** l'appareil de mesure comprend une unité de traitement de données (120), qui est configurée pour déterminer la teneur en humidité compensée en densité du matériau minéral sur la base de la réponse en fréquence du résonateur (100) affectée par le matériau minéral.

4. Appareil de mesure selon la revendication 3, **caractérisé en ce que** l'unité de traitement de données (120) est configurée pour déterminer la teneur en humidité compensée en densité du matériau minéral sur la base d'au moins deux des suivants de la réponse en fréquence : la fréquence de résonance du résonateur (100), la valeur Q de la résonance, et le niveau de signal de la résonance.

5. Appareil de mesure selon la revendication 3, **caractérisé en ce que**
l'unité de traitement de données (120) comprend un ou plusieurs processeurs (700) ; et une ou plusieurs mémoires (702) incluant un code de programme informatique ;
la ou les mémoires (702) et le code de programme informatique sont configurés pour, avec le ou les processeurs (700) amener l'unité de traitement de données (120) au moins à :
déterminer la teneur en humidité du matériau minéral sur la base de la réponse en fréquence du résonateur (100) affectée par le matériau minéral.

6. Appareil de mesure selon la revendication 1, **caractérisé en ce que** l'appareil de mesure comprend deux systèmes d'introduction d'échantillon (200, 202), parmi lesquels un premier système d'introduction d'échantillon (200) est configuré pour délivrer le matériau minéral à un deuxième système d'introduction d'échantillon (202) à un premier débit volumétrique, et le deuxième système d'introduction d'échantillon (202) est configuré pour délivrer le matériau minéral à et à travers la cavité (106) du résonateur (100) à un deuxième débit volumétrique qui est supérieur au premier débit volumétrique.

7. Appareil de mesure selon la revendication 1, **caractérisé en ce que** l'appareil de mesure est configuré pour mesurer un matériau minéral qui comprend les matériaux ferromagnétiques et/ou ferrimagnétiques.

8. Appareil de mesure selon la revendication 1 ou 7, **caractérisé en ce que** l'appareil de mesure est configuré pour mesurer un matériau minéral qui comprend la magnétite et/ou l'hématite.

9. Appareil de mesure selon la revendication 1, **caractérisé en ce que** l'agencement de sondes (108) comprend une deuxième sonde de couplage (302) située à une position (d, α), où d est à une distance radiale d'un axe longitudinal (LA) du résonateur (100) d'une première sonde de couplage (300), et l'angle α est de π/2 entre la première sonde (300) et la deuxième sonde (302).

10. Appareil de mesure selon la revendication 9, **caractérisé en ce que** la deuxième sonde (302) est dans le zéro d'une résonance perturbatrice (402) dans le résonateur (100) afin d'annuler la résonance perturbatrice (402) à une fréquence différente de la fréquence de résonance mesurée (400).

11. Appareil de mesure selon la revendication 1, **caractérisé en ce que** l'agencement de sondes (108) comprend une paire de sondes de couplage (310, 312) situées à une position (1, β), où 1 est à une distance radiale d'un axe longitudinal du résonateur (100) pour la paire de sondes de couplage (310, 312), et l'angle β est de π entre la paire de sondes de couplage (310, 312), et l'appareil de mesure est configuré pour alimenter la paire de sondes de couplage (310, 312) avec un signal qui a la même phase pour les deux d'entre elles afin d'annuler une résonance perturbatrice (402) à une fréquence différente de la fréquence de résonance mesurée (400).

12. Appareil de mesure selon la revendication 1, **caractérisé en ce que**
l'appareil de mesure comprend une conduite (104) attachée au trou (105), la conduite (104) étant configurée pour permettre l'écoulement du matériau minéral à travers l'appareil de mesure ;
l'appareil de mesure comprend une couche (124) en matériau électriquement conducteur sur la plaque de la structure en matériau solide (114) et la conduite (104).

13. Procédé de mesure pour mesurer l'humidité d'un matériau minéral, le procédé comprenant
la délivrance (900) d'énergie électrique à un résonateur (100) d'un appareil de mesure par un agencement de sondes (108), **caractérisée en ce que** le résonateur (100), qui est un résonateur à cavité d'ondes radio et/ou de micro-ondes, est partiellement rempli d'une structure en matériau solide (114), la structure en matériau solide (114) étant une plaque ayant un trou (105) à travers la plaque, le trou (105) étant situé à l'intérieur de la circonférence extérieure (112) et le trou (105) comprenant la cavité (106), et l'agencement de sondes (108) situé à l'intérieur de la structure en matériau solide (114) provoque une résonance électromagnétique à l'intérieur d'une cavité (106) du résonateur (100) en réponse à l'énergie électrique délivrée à celui-ci, la cavité (106) de l'appareil de mesure étant limitée par la structure en matériau solide (114), et la permittivité relative de la structure en matériau solide (114) du résonateur (100) est supérieure à celle de la cavité (106) avec le matériau minéral ;
la création (902), par la structure en matériau solide (114) du résonateur (100) et de la cavité (106), d'une distribution inégale d'un champ magnétique de la résonance entre la cavité (106) et la structure en matériau solide (114), et l'annulation du champ magnétique dans la cavité (106) ayant pour résultat une distribution où le champ magnétique est principalement situé à l'intérieur de la structure en matériau solide (114) du résonateur (100) et moins à l'intérieur de la cavité (106) ;
la délivrance en sortie (904), par l'agencement de sondes (108), d'une réponse en fréquence du résonateur (100) affectée par le matériau minéral à l'intérieur de la cavité (106) pour la détermination de la teneur en humidité du matériau minéral.

14. Procédé de mesure selon la revendication 13, **caractérisé par** la détermination (906) d'une teneur en humidité compensée en densité du matériau minéral sur la base d'au moins deux des suivants de la réponse en fréquence : la fréquence de résonance du résonateur (100), la valeur Q de la résonance, et le niveau de signal de la résonance.
